# FASCICULE DE BREVET EUROPEEN

(11) **EP 1 844 737 B1**
(45) Date de publication et mention de la délivrance du brevet: **03.06.2015**
(21) Numéro de dépôt: 07356047.6
(22) Date de dépôt: 12.04.2007
(51) Int. Cl.: A61F 2/40

(54) **Composant glénoïdien pour prothèse totale d'épaule, jeu de tels composants, et prothèse totale d'épaule comprenant un tel composant**
Glenoidale Komponente für eine Total-Schulterprothese, Kit aus solchen Komponenten, sowie Total-Schulterprothese mit einer solchen Komponente
Glenoid component for complete shoulder prosthesis, set of such components and complete shoulder prosthesis comprising such a component

(30) Priorité: 13.04.2006 FR 0603291
(43) Date de publication de la demande: 17.10.2007
(73) Titulaire: Tornier, 38330 Montbonnot-Saint-Martin (FR)
(72) Inventeur: Ferrari, Irène, 38660 Saint Vincent de Mercuze (FR); Ratron, Yves-Alain, 38000 Grenoble (FR); Ferrand, Lucile, 38330 Montbonnot (FR)
(74) Mandataire: Myon, Gérard Jean-Pierre

(56) Documents cités:
- FR-A1- 2 776 506
- FR-A1- 2 859 099
- US-A- 3 869 730
- US-A- 5 489 310
- US-A1- 2005 261 775

## Description

La présente invention concerne un composant glénoïdien de prothèse totale d'épaule, un jeu de tels composants glénoïdiens, ainsi qu'une prothèse totale d'épaule comprenant un tel composant.

Dans le domaine des prothèses d'épaule il est connu, par exemple de FR-A-2 859 099, d'utiliser un composant glénoïdien, qui comporte tout d'abord une base propre à venir en appui contre la glène du patient. A cet effet, cette base est associée à un organe d'ancrage, destiné à pénétrer profondément dans cette glène, afin de solidariser fermement cette dernière au composant précité. Dans l'agencement de ce document, cet organe d'ancrage présente une forme de quille, à savoir qu'il possède un axe longitudinal principal.

La base présente une surface concave opposée à cette quille, qui est propre à coopérer avec une tête globalement hémisphérique d'un composant huméral de la prothèse d'épaule. Afin d'assurer une implantation satisfaisante, du point de vue anatomique, du composant glénoïdien dans la glène, la quille de ce composant présente une longueur inférieure à celle de la base venant en appui contre la glène. Par ailleurs, la quille est légèrement effilée, à savoir que la longueur de son extrémité libre est inférieure à la longueur que présente cette quille, au niveau de sa zone de raccord avec la base.

On connaît également, par US-A-2005/0261775, une solution alternative dans laquelle l'organe d'ancrage précité est formé par un plot massif. Comme dans le cas de la quille précitée, ce plot présente, au niveau de sa zone de raccord avec la base, des dimensions inférieures à celles-ci, dans un souci anatomique.

Cependant, un tel plot présente des inconvénients, notamment en ce qu'il se révèle relativement invasif à l'égard du patient. En effet, son utilisation nécessite d'enlever une partie importante du stock osseux du patient, ce qui est désavantageux notamment en cas de révision de l'implant.

On connaît enfin, par US-A-3,869,730, qui décrit toutes les caractéristiques du préambule de la revendication 1 annexée, une prothèse d'épaule qui comprend un composant glénoïdien, qui est équipé d'une première plaque diamétrale, ainsi que de deux demi-plaques, s'étendant transversalement par rapport à cette plaque principale. Ces différentes plaques d'ancrage du composant dans la glène ne sont cependant pas anatomiques, étant donné que leurs dimensions sont identiques à celles de la base et que, par conséquent, elles sont susceptibles d'induire une destruction au moins partielle de la glène. A cet égard, on notera que ces plaques ne sauraient être assimilées à une quille d'ancrage, telle qu'évoquée ci-dessus.

Ceci étant précisé, l'invention vise à améliorer les composants glénoïdiens, dont l'organe d'ancrage est formé par une quille présentant un axe longitudinal principal, comme cela est décrit dans FR-A-2 859 099. Elle vise en particulier à proposer un tel composant glénoïdien, dont la résistance mécanique et la stabilité, une fois implanté, sont plus satisfaisants que dans les solutions de l'art antérieur.

A cet effet, elle a pour objet un composant glénoïdien pour prothèse totale d'épaule selon la revendication 1 annexée.

L'invention a également pour objet un jeu de plusieurs composants glénoïdiens selon la revendication 10 annexée.

L'invention a enfin pour objet une prothèse totale d'épaule selon la revendication 12 annexée.

L'invention sera mieux comprise et d'autres avantages de celle-ci apparaîtront plus clairement à la lumière de la description qui va suivre, donnée uniquement à titre d'exemple non limitatif et faite en référence aux dessins annexés, dans lesquels :
- la figure 1 est une représentation schématique de principe d'une prothèse conforme à l'invention, implantée sur un patient ;
- la figure 2 est une vue en perspective du composant glénoïdien appartenant à la prothèse de la figure 1 ;
- la figure 3 est une vue de dessous, illustrant le composant glénoïdien de la figure 2 ; et
- les figures 4 et 5 sont des vues en perspective analogues à la figure 2, illustrant deux variantes de réalisation d'un composant glénoïdien selon l'invention.

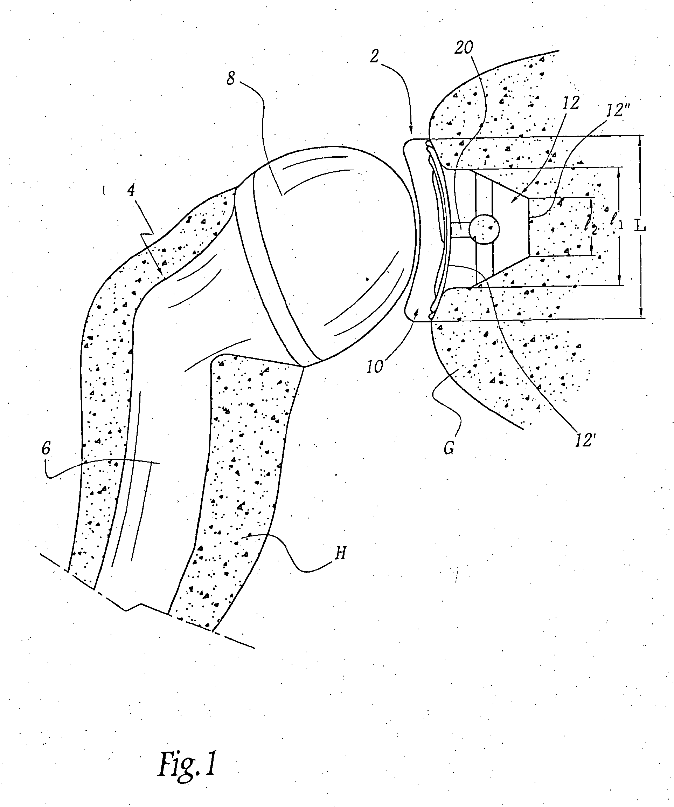

La prothèse totale d'épaule, représentée sur la figure 1, comprend tout d'abord un composant glénoïdien 2, solidarisé à la glène G d'une épaule, ainsi qu'un composant huméral 4, solidarisé à l'humérus H correspondant. Ce composant huméral 4 comprend, de façon connue en soi, une tige 6 destinée à être ancrée dans le canal médullaire de l'humérus H, ainsi qu'une tête hémisphérique 8 définissant une surface convexe en forme de tronçon de sphère.

Le composant glénoïdien 2, qui est réalisé en métal, en matière plastique ou en tout autre matériau biocompatible adapté, comprend tout d'abord une base 10, qui présente une face dite externe 10₁, opposée à la glène. Cette face externe 10₁, de profil concave, est destinée à coopérer de manière habituelle avec la tête 8 du composant huméral 4.

Sur sa face opposée, dite interne 10₂, la base 10 prend appui contre la glène G de l'épaule. A cet effet, cette face interne 10₂ est pourvue de stries 10₃, facilitant l'implantation de la base 10. Cette dernière se prolonge par une quille d'ancrage 12, qui présente une dimension longitudinale, ou longueur, nettement supérieure à sa dimension transversale, ou largeur. A cet effet, on note X-X l'axe principal longitudinal de cette quille 12. On peut affecter à cette quille 12 deux axes supplémentaires, à savoir respectivement Y-Y correspondant à la largeur de la quille, ainsi que Z-Z correspondant à son épaisseur.

Comme cela est connu en soi, par exemple de FR-A-2 859 099, la longueur l₁ de la quille 12, au niveau de sa zone de raccord 12' avec la base 2, est inférieure à la longueur L de la base 2. Par ailleurs, la longueur l₂ de cette quille 12, au niveau de son extrémité libre 12", est inférieure à la longueur 1₁ définie ci-dessus, de sorte que cette quille présente une profil légèrement effilé. Ces différentes longueurs L, l₁ et l₂, qui sont prises selon l'axe principal X-X, sont matérialisées sur la figure 1.

La quille 12 est creusée d'un trou transversal 14, permettant de façon connue en soi de créer un pont de ciment pour l'ancrage du composant, dans le cas où celui-ci est cimenté. Dans le cas où le composant n'est pas cimenté, ce trou est susceptible de permettre la création d'un pont osseux.

Sur chacune des faces latérales de cette quille 12, il est prévu des entailles 16, qui s'étendent à partir des parois du trou 14, en définissant à peu près une forme de T. La fonction de ces entailles est d'assurer une accroche satisfaisante du composant 2 par rapport à la glène G.

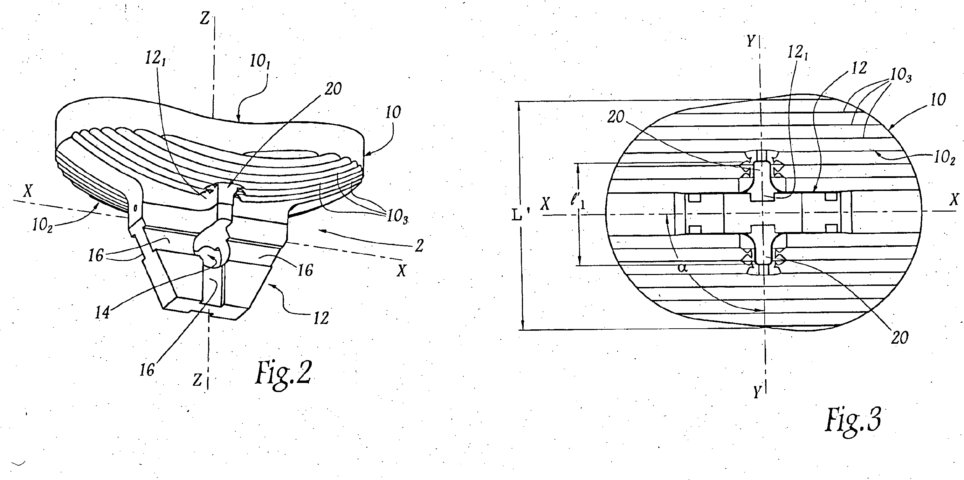

Deux ailerons 20 s'étendent à partir de la quille 12, de façon latérale, en formant un angle α par rapport à l'axe longitudinal X-X, comme le montre notamment la figure 3. Dans l'exemple illustré, cet angle α est voisin de 90° mais, à titre de variante, on peut prévoir qu'il est compris entre 30 et 150°, notamment entre 45 et 135°.

En référence à cette figure 3, on note que les ailerons 20 s'étendent seulement sur une partie de la base 2. En d'autres termes, la distance l'₁ séparant les deux extrémités opposées de ces ailerons, est nettement inférieure à la largeur L' de la base 2, à savoir la dimension de celle-ci selon l'axe Y-Y de ces ailerons.

Par ailleurs, dans l'exemple illustré, on retrouve deux ailerons 20, s'étendant de façon symétrique par rapport à l'axe principal précité X-X. Cependant, à titre de variante, on peut prévoir de faire appel à un unique aileron. A titre de variante supplémentaire, on peut faire appel, sur au moins un des deux côtés du composant, à un nombre d'ailerons supérieur à un. Enfin les dimensions, en particulier les longueurs, des différents ailerons peuvent être différentes les unes des autres de sorte que, en référence à la figure 3, le composant n'est pas symétrique par rapport à l'axe X-X.

En revenant en particulier à la figure 2, chaque aileron 20 se raccorde à la face interne 10₂ de la base 10, ainsi qu'aux parois du trou médian 14. Ainsi, cet aileron s'étend, selon l'axe Z-Z, à peu près sur la moitié de l'épaisseur de la quille 12. Cependant, à titre de variante, l'épaisseur de l'aileron peut être plus importante. Dans cette optique, ce dernier s'étend par exemple jusqu'au bord inférieur de la quille, en présentant une forme effilée en direction de ce bord inférieur, notamment de type pyramidale, de manière à épouser la forme interne de la glène anatomique.

Par ailleurs, chaque aileron 20 fait saillie, par rapport à la quille, à partir d'une région médiane 12₁ de cette dernière. Cette région médiane 12₁ est définie en référence à l'axe principal longitudinal X-X de la quille 12, auquel il est fait référence ci-dessus. A titre de variante non représentée, on notera que chaque aileron peut être creusé d'un trou transversal, analogue à celui 14 ménagé dans la quille, de manière à permettre la création d'un pont de ciment ou d'un pont osseux.

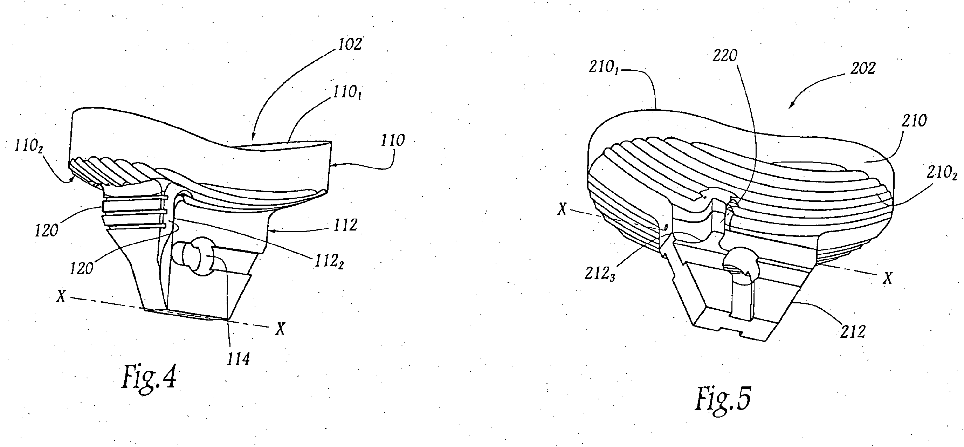

Les figures 4 et 5 illustrent deux variantes de réalisation de l'invention, qui concernent la position relative des ailerons et de la quille.

Sur la figure 4, les éléments mécaniques analogues à ceux des figures 1 à 3 y sont affectés des mêmes numéros de référence, augmentés de 100. La différence entre le composant 102 de cette figure 4 et celui 2 des figures 1 à 3, réside dans le fait que les ailerons 120 font saillie par rapport à la quille 112, à partir d'une région d'extrémité 112₂ de celle-ci.

Cette région d'extrémité est définie, comme ci-dessus pour la région médiane, en référence à l'axe longitudinal principal X-X de la quille 112. On notera que, dans cette première variante de réalisation, l'angle formé par les ailerons avec cet axe principal X-X est également voisin de 90°.

Sur la figure 5, les éléments mécaniques analogues à ceux des figures 1 à 3 y sont affectés des mêmes numéros de référence, augmentés de 200. La différence entre le composant glénoïdien 202 de cette figure 5 et celui 2 des figures 1 à 3, réside dans le fait que les ailerons 220 font saillie par rapport à la quille 212, à partir d'une région intermédiaire 212₃ de celle-ci.

Comme dans ce qui précède, cette région intermédiaire est définie en faisant référence à l'axe longitudinal principal X-X de la quille 212. En d'autres termes, cette région intermédiaire 212₃ est située entre les régions respectivement médiane et d'extrémité, définies ci-dessus. De façon analogue aux deux premiers modes de réalisation, les deux ailerons 220 forment sensiblement un angle droit, par rapport à l'axe principal X-X de la quille 212.

Comme on l'a vu dans ce qui précède, la position relative des ailerons et de la quille est variable, à savoir que ces ailerons peuvent s'étendre à partir de différents emplacements, le long de la quille. Dans ces conditions, l'invention permet également de fournir un jeu de composants glénoïdiens, dont les ailerons présentent des positions différentes les uns par rapport aux autres.

Dans cette optique, un tel jeu comprend par exemple au moins un composant dit médian 2, au moins un composant dit d'extrémité 102, ainsi qu'au moins un composant dit intermédiaire 202. En fonction de l'anatomie du patient, le chirurgien est ainsi à même d'implanter le composant glénoïdien approprié 2, 102 ou 202 lors de l'opération. On peut également envisager d'utiliser d'autres composants, non représentés sur les figures, pour lesquels le ou chaque aileron présente une position et/ou une orientation angulaire et/ou des dimensions différentes de celles décrites ci-dessus.

L'invention permet de réaliser les objectifs précédemment mentionnés.

En effet, la présence d'au moins un aileron transversal confère plusieurs avantages au composant glénoïdien qui en est équipé.

Ainsi, grâce à l'invention, la tenue en bascule du composant glénoïdien est améliorée. Ceci permet en particulier d'éviter, dans une large mesure, tout déscellement de ce composant par rapport à la glène.

Par ailleurs, la rigidité de la base du composant est augmentée, par rapport aux solutions de l'art antérieur. Ceci permet d'éviter que ne se produisent des micromouvements, du fait de l'amélioration de la tenue primaire de l'ensemble du composant.

Enfin, grâce à la présence du ou de chaque aileron, l'implant présente une résistance au cisaillement qui est plus importante que dans l'état de la technique connu.

## Revendications

1. Composant glénoïdien (2 ; 102 ; 202) pour prothèse totale d'épaule, comprenant une base (10 ; 110 ; 210) propre à prendre appui, par une première face (102 ; 110₂ ; 210₂), sur la glène (G) d'une épaule, cette base étant propre à coopérer, par une autre face (10₁ ; 110₁ ; 210₁), avec un composant huméral (4) de ladite prothèse d'épaule, ce composant glénoïdien comprenant également une quille (12 ; 112 ; 212) d'ancrage dans la glène (G), qui s'étend à partir de ladite base et qui présente un axe longitudinal principal (X-X), ce composant glénoïdien comprenant également au moins un aileron transversal (20 ; 120 ; 220), en référence audit axe longitudinal principal (X-X), le ou chaque aileron transversal s'étendant latéralement à partir de la quille (12 ; 112 ; 212), **caractérisé en ce que** la dimension (11) de la quille (12) selon son axe longitudinal principal (X-X), au niveau de la zone de raccord (12') avec la base (10), est inférieure à la dimension (L) de cette base (10) selon cet axe longitudinal principal, et **en ce que** le ou chaque aileron transversal (20 ; 120 ; 220) s'étend sur à peu près la moitié de l'épaisseur de la quille (12 ; 112 ; 212).

2. Composant glénoïdien selon la revendication 1, **caractérisé en ce que** le ou chaque aileron transversal (20 ; 120 ; 220) forme, avec l'axe longitudinal principal (X-X) de la quille (12 ; 112 ; 212), un angle α compris entre 30 et 150°, de préférence entre 45 et 135° ; notamment égal à 90°.

3. Composant glénoïdien selon la revendication 1 ou 2, **caractérisé en ce que** le ou chaque aileron transversal (20 ; 120 ; 220) est raccordé à la base (10 ; 110 ; 210).

4. Composant glénoïdien selon l'une quelconque des revendications précédentes, **caractérisé en ce que** ce composant est pourvu de deux ailerons transversaux (20 ; 120 ; 220), s'étendant de façon symétrique par rapport audit axe longitudinal principal (X-X).

5. Composant glénoïdien selon l'une des revendications 1 à 4, **caractérisé en ce que** le ou chaque aileron transversal (20) s'étend à partir d'une région médiane (12₁) de la quille (12), en référence audit axe longitudinal principal.

6. Composant glénoïdien selon l'une des revendications 1 à 4, **caractérisé en ce que** le ou chaque aileron transversal (120) s'étend à partir d'une région d'extrémité (112₂) de la quille (112), en référence audit axe longitudinal principal.

7. Composant glénoïdien selon l'une des revendications 1 à 4, **caractérisé en ce que** le ou chaque aileron transversal (20) s'étend à partir d'une région intermédiaire (212₃) de la quille (212), en référence audit axe longitudinal principal.

8. Composant glénoïdien selon l'une quelconque des revendications précédentes, **caractérisé en ce que** la dimension (l₂) de la quille (12) selon son axe longitudinal principal (X-X), au niveau de son extrémité libre (12"), est inférieure à la dimension (l₁) de cette quille au niveau de sa zone de raccord avec la base (10).

9. Composant glénoïdien selon l'une quelconque des revendications précédentes, **caractérisé en ce que** le ou chaque aileron transversal (20 ; 120 ; 220) s'étend uniquement sur une partie de la base (10 ; 110 ; 210), selon l'axe principal (Y-Y) du ou de chaque aileron.

10. Jeu de plusieurs composants glénoïdiens selon l'une quelconque des revendications précédentes, dans lequel la position du ou de chaque aileron (20 ; 120 ; 220) par rapport à la quille (12 ; 112 ; 212) est variable, cette position étant en prise en référence à la direction longitudinale principale de la quille.

11. Jeu selon la revendication 10, **caractérisé en ce que** ce jeu comprend au moins un composant glénoïdien (2) selon la revendication 5, au moins un composant glénoïdien (102) selon la revendication 6 et au moins un moins un composant glénoïdien (202) selon la revendication 7.

12. Prothèse totale d'épaule, comprenant un composant glénoïdien (2 ; 102 ; 202) suivant l'une quelconque des revendications 1 à 9, ainsi qu'un composant huméral (4) propre à coopérer avec ledit composant glénoïdien.

## Patentansprüche

1. Glenoidkomponente (2; 102; 202) für eine Total-Schulterprothese, eine Basis (10; 110; 210) umfassend, die geeignet ist, sich mit einer ersten Fläche (10₂; 110₂; 210₂) auf der Gelenkpfanne (G) einer Schulter abzustützen, wobei diese Basis geeignet ist, über eine andere Fläche (10₁; 110₁; 210₁) mit einer Oberarmkomponente (4) der Schulterprothese zusammenzuarbeiten, wobei diese Glenoidkomponente gleichfalls einen Kiel (12; 112; 212) zur Verankerung in der Gelenkpfanne (G) umfasst, der sich von der Basis erstreckt und der eine Hauptlängsachse (X-X) aufweist, wobei diese Glenoidkomponente gleichfalls mindestens eine Querrippe (20; 120; 220) bezogen auf die Hauptlängsachse (X-X) umfasst, wobei die oder jede Querrippe sich seitlich von dem Kiel (12; 112; 212) erstreckt, **dadurch gekennzeichnet, dass** die Abmessung (l₁) des Kiels (12) gemäß seiner Hauptlängsachse (X-X) an der Verbindungzone (12') mit der Basis (10) kleiner ist als die Abmessung (L) dieser Basis (10) gemäß dieser Hauptlängsachse und dass die oder jede Querrippe (20; 120; 220) sich ungefähr über die Hälfte der Dicke des Kiels (12; 120; 212) erstreckt.

2. Glenoidkomponente nach Anspruch 1, **dadurch gekennzeichnet, dass** die oder jede Querrippe (20; 120; 220) mit der Hauptlängsachse (X-X) des Kiels (12; 112; 212) einen Winkel α bildet, der zwischen 30 und 150°, vorzugsweise zwischen 45 und 135° liegt, insbesondere gleich 90° ist.

3. Glenoidkomponente nach Anspruch 1 oder 2, **dadurch gekennzeichnet, dass** die oder jede Querrippe (20; 120; 220) mit der Basis (10; 110; 210) verbunden ist.

4. Glenoidkomponente nach einem beliebigen der vorhergehenden Ansprüche, **dadurch gekennzeichnet, dass** diese Komponente mit zwei Querrippen (20; 120; 220) versehen ist, die sich symmetrisch in Bezug auf die Hauptlängsachse (X-X) erstrecken.

5. Glenoidkomponente nach einem beliebigen der Ansprüche 1 bis 4, **dadurch gekennzeichnet, dass** die oder jede Querrippe (20) sich von einer Mittelregion (12₁) des Kiels (12) bezogen auf die Hauptlängsachse erstreckt.

6. Glenoidkomponente nach einem beliebigen der Ansprüche 1 bis 4, **dadurch gekennzeichnet, dass** die oder jede Querrippe (120) sich von einem Endbereich (112₂) des Kiels (112) bezogen auf die Hauptlängsachse erstreckt.

7. Glenoidkomponente nach einem beliebigen der Ansprüche 1 bis 4, **dadurch gekennzeichnet, dass** die oder jede Querrippe (20) sich von einem Mittelbereich (212₃) des Kiels (212) bezogen auf die Hauptlängsachse erstreckt.

8. Glenoidkomponente nach einem beliebigen der vorhergehenden Ansprüche, **dadurch gekennzeichnet, dass** die Abmessung (l₂) des Kiels (12) gemäß seiner Hauptlängsachse (X-X) an seinem freien Ende (12") kleiner ist als die Abmessung (l₁) dieses Kiels an seiner Verbindungszone mit der Basis (10).

9. Glenoidkomponente nach einem beliebigen der vorhergehenden Ansprüche, **dadurch gekennzeichnet, dass** die oder jede Querrippe (20; 120; 220) sich nur über einen Teil der Basis (10; 110; 210) gemäß der Hauptachse (Y-Y) des oder jeder Rippe erstreckt.

10. Satz von mehreren Glenoidkomponenten nach einem beliebigen der vorhergehenden Ansprüche, bei dem die Position der oder jeder Rippe (20; 120; 220) in Bezug auf den Kiel (12; 112; 212) variabel ist, wobei diese Position im Eingriff bezogen auf die Hauptlängsrichtung des Kiels ist.

11. Satz nach Anspruch 10, **dadurch gekennzeichnet, dass** dieser Satz mindestens eine Glenoidkomponente (2) nach Anspruch 5, mindestens eine Glenoidkomponente (102) nach Anspruch 6 ,und mindestens eine Glenoidkomponente (202) nach Anspruch 7 umfasst.

12. Total-Schulterprothese, eine Glenoidkomponente (2; 102; 202) nach einem beliebigen der Ansprüche 1 bis 9 sowie eine Oberarmkomponente (4) umfassend, die geeignet ist, mit der Glenoidkomponente zusammenzuarbeiten.

## Claims

1. Glenoid component (2; 102; 202) for a total shoulder prosthesis, comprising a base (10; 110; 210) adapted to bear against, by means of a first face (10₂; 110₂; 210₂), the glenoid bone (G) of a shoulder, that base being adapted to co-operate, by means of another face (10₁; 110₁; 210₁), with a humeral component (4) of said shoulder prosthesis, said glenoid component also comprising a keel (12; 112; 212) for anchoring in the glenoid bone (G), which extends starting from said base and which has a main longitudinal axis (X-X), said glenoid component also comprising at least one fin (20; 120; 220) which is transverse relative to said main longitudinal axis (X-X), the or each transverse fin extending laterally starting from the keel (12; 112; 212), **characterised in that** the dimension (l₁) of the keel (12) along its main longitudinal axis (X-X) at the level of the zone of connection (12') to the base (10) is less than the dimension (L) of said base (10) along said main longitudinal axis; and **in that** the or each transverse fin (20; 120; 220) extends over approximately half the thickness of the keel (12; 112; 212).

2. Glenoid component according to claim 1, **characterised in that** the or each transverse fin (20; 120; 220) forms, with the main longitudinal axis (X-X) of the keel (12; 112; 212), an angle α of from 30 to 150°, preferably from 45 to 135°; especially of 90°.

3. Glenoid component according to claim 1 or 2, **characterised in that** the or each transverse fin (20; 120; 220) is connected to the base (10; 110; 210).

4. Glenoid component according to any one of the preceding claims, **characterised in that** said component is provided with two transverse fins (20; 120; 220) extending symmetrically with respect to said main longitudinal axis (X-X).

5. Glenoid component according to one of claims 1 to 4, **characterised in that** the or each transverse fin (20) extends starting from a central region (12₁) of the keel (12) with reference to said main longitudinal axis.

6. Glenoid component according to one of claims 1 to 4, **characterised in that** the or each transverse fin (120) extends starting from an end region (112₂) of the keel (112) with reference to said main longitudinal axis.

7. Glenoid component according to one of claims 1 to 4, **characterised in that** the or each transverse fin (20) extends starting from an intermediate region (212₃) of the keel (212) with reference to said main longitudinal axis.

8. Glenoid component according to any one of the preceding claims, **characterised in that** the dimension (l₂) of the keel (12) along its main longitudinal axis (X-X) at the level of its free end (12") is less than the dimension (l₁) of said keel at the level of its zone of connection to the base (10).

9. Glenoid component according to any one of the preceding claims, **characterised in that** the or each transverse fin (20; 120; 220) extends solely over a portion of the base (10; 110; 210) along the main axis (Y-Y) of the or each fin.

10. Set of a plurality of glenoid components according to any one of the preceding claims, wherein the position of the or each fin (20; 120; 220) with respect to the keel (12; 112; 212) is variable, that position being taken with reference to the main longitudinal direction of the keel.

11. Set according to claim 10, **characterised in that** said set comprises at least one glenoid component (2) according to claim 5, at least one glenoid component (102) according to claim 6, and at least one glenoid component (202) according to claim 7.

12. Total shoulder prosthesis, comprising a glenoid component (2; 102; 202) according to any one of claims 1 to 9, and also a humeral component (4) adapted to co-operate with said glenoid component.
